# EUROPEAN PATENT SPECIFICATION

(11) **EP 0 585 359 B1**
(45) Date of publication and mention of the grant of the patent: **18.03.1998**
(21) Application number: 92912258.8
(22) Date of filing: 06.05.1992
(51) Int. Cl.: A61M 25/01, A61B 5/00

(54) **CATHETER GUIDE WIRE**
KATHETERFUEHRUNGSDRAHT
FIL DE GUIDAGE DE CATHETER

(30) Priority: 07.05.1991 US 696585
(43) Date of publication of application: 09.03.1994
(60) Divisional application: 97114538.8
(73) Proprietor: TARGET THERAPEUTICS, INC., Fremont, CA 94537-5120 (US)
(72) Inventor: SEPETKA, Ivan, Redwood City, CA 94063 (US)
(74) Representative: Price, Nigel John King
(86) International application number: US9203896
(87) International publication number: WO9219151

(56) References cited:
- EP-A- 0 274 412
- EP-A- 0 405 823
- EP-A- 0 466 437
- US-A- 4 003 369
- US-A- 4 884 579
- US-A- 4 964 409

## Description

This invention is in the general field of surgical instruments and relates specifically to guide wires that are used in cardiovascular and endovascular procedures to facilitate the placement of catheters within the vasculature of patients.

The general procedure for placing catheters within vessels is to track a guide wire through the vessel to the desired position and advance the catheter over the guide wire. Guide wires are required because the catheters themselves do not have sufficient column strength or torsional strength to be able to be tracked or steered through the vessel. See, for instance, US-A-4,884,579.

Several types of guide wires for use in catheter placement have been proposed. The simplest type of wire has a preferred diameter of between about 0.20-1.0 mm. The distal end of the wire may be provided with a bent tip which can be oriented, by means of a guide structure at the proximal end, to guide the wire along a selected vascular path. Ideally, torque transmission should be controlled, such that a selected wire rotation at the wire's proximal end produces a corresponding rotation of the distal end. Further, radiopacity is desired such that a physician may see over the entire vasculature accessed by the guide wire.

The present invention is an improvement on the guide wire assembly described in US-A-4,884,579 upon which is based the preamble of claim 1 of Claim Set A. The prior invention describes a catheter guide wire with three sections with progressively greater flexibility and sliding properties: 1. A semi-rigid, torqueable proximal wire section that is between about 50-250 cm in length, formed of a proximal wire core segment having an outer diameter of between about 0.25-1.0 mm; 2. A more flexible intermediate section that has a length between about 20-60 cm and is formed from an intermediate wire-core segment having a reduced diameter of between about 0.10-0.50 mm, and a low-friction, flexible polymer tube covering which encases the intermediate core segment; and 3. A most distal end section with a length between about 1-10 cm and formed from a distal wire core segment having a reduced diameter of between about 0.05-0.15 mm, and a flexible sleeve covering the distal end segment and providing column strength thereto.

EP-A-0 274 412 discloses a steerable guidewire having an elongate main wire having a tapered distal portion and a helical coil mounted about the distal portion. The distal end of the coil extends beyond the distal tip of the tapered distal portion of the main wire. An inner helical coil is disposed within the outer coil and is secured at its proximal end to the distal tip of the tapered distal portion of the main wire and at its distal end to the distal end of the outer coil. The device does not have a separate safety wire; the inner coil serves as the sole safety connection between the main wire and the outer coil. The omission of the conventional safety wire and the use of the dual coil construction provides a tip which is equally flexible in all directions.

### Designations of AT, BE, CH, DK, ES, GR, LI, IT, LU, MC, SE

For the designations of Austria, Belgium, Switzerland, Denmark, Spain, Greece, Liechtenstein, Italy, Luxembourg, Monaco and Sweden, according to a first aspect of the present invention there is provided a catheter guidewire in accordance with claim 1 of the accompanying claim Set A. This catheter guidewire is for use within a patient's vasculature and comprises in combination:
(a) a semi-rigid, torqueable proximal wire section,
(b) a more flexible intermediate section formed from an intermediate wire core segment having a flexible polymer tube covering which encases the intermediate wire core segment, and
(c) a most flexible distal end section formed from a distal wire core segment with a helical coil covering the distal end segment and providing column strength thereto,
   characterised in that the guidewire further comprises a radioopaque helical ribbon coil wrapped about the intermediate wire core segment between the intermediate wire core segment and the flexible polymer tube covering and extending along the intermediate wire core segment only whereby to improve torque transmission along the guide wire and to increase radioopacity of the intermediate section over that of the bare intermediate core segment without substantially increasing its stiffness.

Relative to the guidewire disclosed in US-A-4,884, 579, the guidewire of the first aspect of the present invention has an improved torque transmission characteristic and improved radioopacity.

### Designations of DE, FR, GB, NL

EP-A-0 466 437, which claims a priority date of 9th July 1990, was filed on 8th July 1991 and was published on 15th January 1992, is admissible as prior art against the present application under Article 54(3) EPC only, and then only for the common designations of Germany, France, the United Kingdom and the Netherlands.

EP-A-0 466 437 discloses an elongated flexible guide wire for positioning within a patient, comprising:
a) a flexible wire core having a first diameter portion extending from a proximal end to a distal region of the guide wire, and that tapers uniformly along a first tapered portion of said wire core to a second lesser diameter portion shorter than said first diameter portion and then again tapers uniformly along a second tapered portion to a flattened distal portion of said wire core;
b) a flexible coiled wire spring that surrounds the wire core and is attached to said core along the length of the second lesser diameter portion of the flexible wire core and that separates from the core wire at a proximal portion of the second tapered portion, extends along the second tapered and flattened distal portions, and is attached to the flattened distal portion of said wire core at a distal end of the guide wire; and
c) a polymer coating covering all but an extreme proximal portion of the first diameter portion of said wire core.

According to a second aspect of the present invention there is provided, for the designations of Germany, France, the United Kingdom and the Netherlands only, a catheter guidewire in accordance with claim 1 of the accompanying claim Set B. This catheter guidewire is for use within a patient's vasculature and comprises in combination:
(a) a semi-rigid, torqueable proximal wire section,
(b) a more flexible intermediate section formed from an intermediate wire core segment having a flexible polymer tube covering which encases the intermediate wire core segment, and
(c) a most flexible distal end section formed from a distal wire core segment with a helical coil covering the distal end segment and providing column strength thereto,
   wherein the guidewire further comprises a radioopaque helical ribbon coil wrapped about the intermediate wire core segment between the intermediate wire core segment and the flexible polymer tube covering and extending along the intermediate wire core segment only whereby to improve torque transmission along the guide wire and to increase radioopacity of the intermediate section over that of the bare intermediate core segment without substantially increasing its stiffness, and
   wherein the ribbon coil is formed from a radioopaque metal selected from the group consisting of platinum, gold, tungsten and their alloys.

Embodiments of guidewires in accordance with the present invention will now be described, by way of example only, with reference to the accompanying drawings. In the drawings:
Fig. 1 shows fragmentary portions of a guide wire constructed according to one embodiment of the invention.
Fig. 2 shows fragmentary portions of a guide wire constructed according to another embodiment of the invention.

Like parts are referred to by the same reference numerals in the figures.

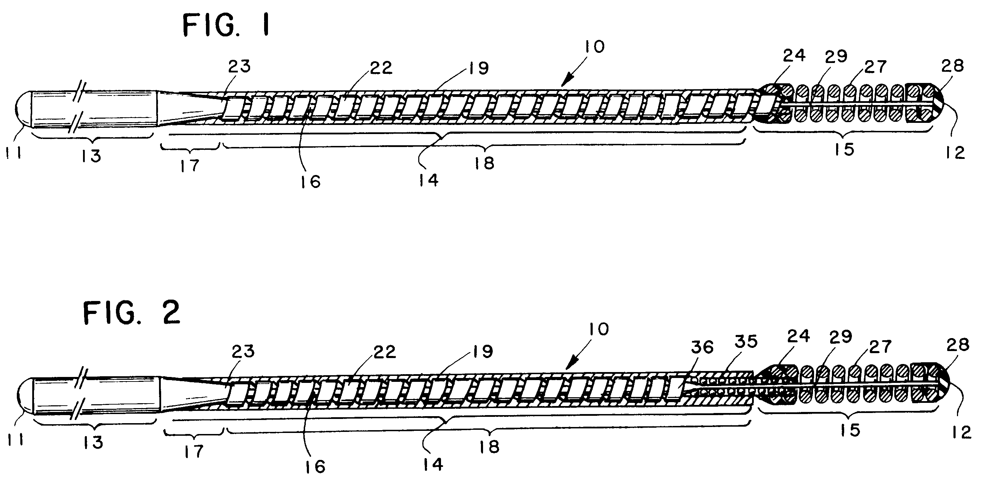

Fig. 1 shows a guide wire generally designated 10, constructed according to one embodiment of the invention. The wire is a flexible torqueable wire having an overall length of about 70-300 cm between its proximal and distal ends 11 and 12, respectively, and a maximum outer diameter of between about 0.20-1.0 mm. The major portion of the wire is a flexible proximal section 13 whose overall length ranges from about 50-250 cm. This section is followed by a more flexible intermediate section 14 having a length between about 20-60 cm and a most flexible distal end section 15 whose length is between about 1-10 cm.

A wire core 16 in the guide wire 10 is formed of a flexible, torqueable wire filament material, such as stainless steel. The diameter of the wire core, at its maximum, is between about 0.20-1.0 mm. The segment of the core forming proximal section 13 of guide wire 10 has a substantially uniform diameter along its length, and corresponds to the maximum diameter of the core, i.e., between 0.20-1.0 mm.

Within the intermediate section 14 of the wire, the core is tapered from the proximal-section diameter down to a reduced diameter which is preferably about 0.10-0.50 mm and between about 10%-50% of the diameter of the core's proximal segment 13. Thus, for example, where the proximal section core diameter is 0.46 mm, the core tapers to a minimum of between about 0.05-0.23 mm. The length of tapered segment 17 is typically between about 10%-50% that of reduced-diameter segment 18, and the two segments together make up the length of the intermediate wire section 14, i.e., about 20-60 cm.

The wire core 16 of intermediate section 14 is covered along its length by a flexible polymer covering 19. The major function of covering 19 is to provide a low-friction surface along intermediate section 14, and more particularly, a surface which has less friction than the surface of adjacent distal segment 15 and proximal segment 13 (the wire core itself). Covering 19 preferably also functions to provide column support to the reduced-diameter core of the intermediate section, 18, to reduce the tendency of this section to buckle under axial compression.

Covering 19 is preferably formed of a polymer, such as TEFLON™, polyolefin, or polyurethane which can be bonded or otherwise tightly affixed to the core wire, and which itself has a low-friction surface, or can be coated with a low-friction surface. Other suitable coverings include a tube formed from virtually any polymer having exposed hydrogens, such as polyester, polyolefins, polycarbonate, polyvinylchloride, latex or silicon rubber, polystyrene, and polyacrylics, and a surface coating formed of a highly hydrophilic, low-friction polymer, such as polyvinylpyrrolidone (PVP), polyethyleneoxide, or polyhydroxyethylmethacrylate (polyHEMA) or copolymers thereof.

Beneath polymer covering 19, a ribbon of radiopaque metal 22, such as platinum, gold, tungsten, or their alloys is wound around the wire core 16. As shown, the ribbon coil 22 extends from tapered segment 17 of intermediate section 14 at junction 23, to the distal junction 24 of intermediate section 14. The ribbon coil 22 has a thickness of about 0.015 to 0.050 mm, preferably about 0.025 mm and a width of about 0.050 to 0.130 mm, preferably about 0.075 mm. There are approximately 5 to 15 complete turns of ribbon per millimeter of wire core, and preferably about 10 complete turns of ribbon per millimeter of wire core.

The distal section 15 of guide wire 10 is fully or partially encased in flexible sleeve 27. Sleeve 27 shown in Fig. 1 is a soft, flexible helical coil which is formed conventionally, e.g., as a winding of radiopaque wire strand, such as platinum, gold, or tungsten strand. The wire strand has a diameter of about 0.050 to 0.100 mm and preferably about 0.075 mm. As shown, sleeve 27 extends from junction 24 to distal end 12 of guide wire 10. Attachment of the sleeve 27 to wire core 16 is preferably by two or three solder or weld joints, one at proximal junction 24 and a second at rounded distal junction 28.

In addition to providing a mechanism for wire bending near wire tip 12, sleeve 27 also gives distal section 15 of guide wire 10 increased column strength (in the axial direction), and reduces the chance of buckling in this section with axial compression. At the same time, the combined flexibility of reduced diameter core 29 and sleeve 27 are compatible with a series of sharp bends, as the wire is moved through a patient's vasculature. Rounded joint 28 at the end of guide wire 10 acts to shield vessel walls from the sharp end of wire core 16. Further, the distal section of the wire, 15, with the associated sleeve 27, provides the section with a higher frictional coefficient than that of the adjacent intermediate section, 14. The higher-friction surface in this distal section, 15, functions specifically, during a catheter placement operation, to help anchor distal section 15 against a vessel wall at a vessel junction.

Distal wire core 29 has a substantially uniform cross-section. The core may be cylindrical with diameter of between 0.05 and 0.15 mm or flattened with a rectangular cross-section dimensioned 0.025 mm by 0.075 mm. The wire core has a tapered section at junction 24 that covers between about 10-50% of the core's distal segment.

Fig. 2 is another embodiment of the invention that is essentially the same as Fig. 1 except for the replacement of a portion of helical ribbon coil 22 with inner wire coil 35. At the distal end of helical ribbon coil 22, there is a soft flexible helical coil 24 which is formed conventionally, e.g., as a winding of radiopaque wire strand, such as platinum, gold or tungsten. As shown, coil 35 extends from helical ribbon coil 22 at junction 36 to the proximal end of distal segment 15 at junction 24. This inner coil 35 serves as an anchor point for the distal end of flexible helical coil 22 and also as an anchor point for the polymer covering 19 on intermediate core section 14.

## Claims (Claims for the following Contracting State(s): AT, BE, CH, DK, ES, GR, LI, IT, LU, MC, SE)

1. A catheter guidewire (10) for use within a patient's vasculature, comprising in combination:
(a) a semi-rigid, torqueable proximal wire section (13),
(b) a more flexible intermediate section (14) formed from an intermediate wire core segment having a flexible polymer tube covering (19) which encases the intermediate wire core segment, and
(c) a most flexible distal end section (15) formed from a distal wire core segment with a helical coil (27) covering the distal end segment and providing column strength thereto,
characterised in that the guidewire further comprises a radioopaque helical ribbon coil (22) wrapped about the intermediate wire core segment between the intermediate wire core segment and the flexible polymer tube covering (19) and extending along the intermediate wire core segment only whereby to improve torque transmission along the guide wire and to increase radioopacity of the intermediate section (14) over that of the bare intermediate core segment without substantially increasing its stiffness.

2. The guidewire of claim 1, wherein the ribbon coil (22) is formed from a radioopaque metal selected from the group consisting of platinum, gold, tungsten and their alloys.

3. The guidewire of claim 1 or claim 2, wherein the ribbon coil (22) is a tightly wound coil with a thickness of about 0.015 to 0.050 mm and a width of about 0.050 to 0.130 mm, which extends from the proximal wire section (13) of the guide wire to the junction of the distal wire core segment.

4. The guidewire of any one of the preceding claims, wherein:
(a) the flexible, torqueable proximal wire section (13) has an outer diameter of between about 0.25-1.00 mm, and
(b) the intermediate wire core segment has a reduced diameter of between about 0.10-0.50 mm, and
(c) the distal wire core segment has a reduced cross-sectional area.

5. The guidewire of claim 4, wherein the distal wire core segment is cylindrical and has a diameter of between about 0.05 and 0.15 mm.

6. The guidewire of claim 4, wherein the distal wire core segment is rectangular with cross-sectional dimensions of 0.025 mm by 0.075 mm.

7. The guidewire of any one of claims 1 to 3, wherein:
(a) the flexible, torqueable proximal wire section (13) has an outer diameter of between about 0.25-0.50 mm, and
(b) the intermediate wire core segment has a reduced diameter of between about 0.10-0.20 mm, and
(c) the distal wire core segment has a reduced diameter of between about 0.05-0.13 mm.

8. The guidewire of any one of the preceding claims, wherein a platinum helical coil (35) wrapped around the proximal end of the distal section (15) serves as an anchor point for the proximal end of the distal helical coil (27) and the anchor point for the distal end of the flexible polymer tube covering (19) which encases the intermediate wire core segment and the helical ribbon coil (22).

## Claims (Claims for the following Contracting State(s): DE, FR, GB, NL)

1. A catheter guidewire (10) for use within a patient's vasculature, comprising in combination:
(a) a semi-rigid, torqueable proximal wire section (13),
(b) a more flexible intermediate section (14) formed from an intermediate wire core segment having a flexible polymer tube covering (19) which encases the intermediate wire core segment, and
(c) a most flexible distal end section (15) formed from a distal wire core segment with a helical coil (27) covering the distal end segment and providing column strength thereto,
wherein the guidewire further comprises a radioopaque helical ribbon coil (22) wrapped about the intermediate wire core segment between the intermediate wire core segment and the flexible polymer tube covering (19) and extending along the intermediate wire core segment only whereby to improve torque transmission along the guide wire and to increase radioopacity of the intermediate section (14) over that of the bare intermediate core segment without substantially increasing its stiffness, and wherein the ribbon coil (22) is formed from a radioopaque metal selected from the group consisting of platinum, gold, tungsten and their alloys.

2. The guidewire of claim 1, wherein the ribbon coil (22) is a tightly wound coil with a thickness of about 0.015 to 0.050 mm and a width of about 0.050 to 0.130 mm, which extends from the proximal wire section (13) of the guide wire to the junction of the distal wire core segment.

3. The guidewire of any one of the preceding claims, wherein:
(a) the flexible, torqueable proximal wire section (13) has an outer diameter of between about 0.25-1.00 mm, and
(b) the intermediate wire core segment has a reduced diameter of between about 0.10-0.50 mm, and
(c) the distal wire core segment has a reduced cross-sectional area.

4. The guidewire of claim 3, wherein the distal wire core segment is cylindrical and has a diameter of between about 0.05 and 0.15 mm.

5. The guidewire of claim 3, wherein the distal wire core segment is rectangular with cross-sectional dimensions of 0.025 mm by 0.075 mm.

6. The guidewire of either of claims 1 and 2, wherein:
(a) the flexible, torqueable proximal wire section (13) has an outer diameter of between about 0.25-0.50 mm, and
(b) the intermediate wire core segment has a reduced diameter of between about 0.10-0.20 mm, and
(c) the distal wire core segment has a reduced diameter of between about 0.05-0.13 mm.

7. The guidewire of any one of the preceding claims, wherein a platinum helical coil (35) wrapped around the proximal end of the distal section (15) serves as an anchor point for the proximal end of the distal helical coil (27) and the anchor point for the distal end of the flexible polymer tube covering (19) which encases the intermediate wire core segment and the helical ribbon coil (22).

## Patentansprüche (Patentansprüche für folgende(n) Vertragsstaat(en): AT, BE, CH, DK, ES, GR, LI, IT, LU, MC, SE)

1. Katheter-Führungsdraht (10) zur Verwendung in dem Gefäßsystem eines Patienten, wobei der Führungsdraht in Kombination folgendes umfaßt:
(a) einen halbstarren drehbaren proximalen Drahtabschnitt (13),
(b) einen flexibleren Zwischenabschnitt (14), der aus einem Zwischendrahtkernsegment gebildet ist, das einen flexiblen Polymerschlauchmantel (19) aufweist, der das Zwischendrahtkernsegment ummantelt, und
(c) einen am meisten flexiblen distalen Endabschnitt (15), der aus einem distalen Drahtkernsegment mit einer schraubenförmigen Spule (27) gebildet ist, die das distale Endsegment abdeckt und diesem eine Knickfestigkeit verleiht,
dadurch gekennzeichnet, daß der Führungsdraht außerdem eine strahlenundurchlässige schraubenförmige Bandspule (22) umfaßt, die um das Zwischendrahtkernsegment zwischen dem Zwischendrahtkernsegment und dem flexiblen Polymerschlauchmantel (19) gewickelt ist und sich nur entlang dem Zwischendrahtkernsegment erstreckt, um die Drehmomentübertragung entlang dem Führungsdraht zu verbessern und um die Strahlenundurchlässigkeit des Zwischenabschnitts (14) gegenüber der des freiliegenden Zwischenkernsegments zu erhöhen, ohne daß dessen Steifigkeit wesentlich erhöht wird.

2. Führungsdraht nach Anspruch 1, bei dem die Bandspule (22) aus einem strahlenundurchlässigen Metall gebildet ist, das aus der Gruppe ausgewählt wird, die aus Platin, Gold, Wolfram und deren Legierungen besteht.

3. Führungsdraht nach Anspruch 1 oder 2, bei dem die Bandspule (22) eine straff gewickelte Spule mit einer Dicke von etwa 0,015 bis 0,050 mm und einer Breite von etwa 0,050 bis 0,130 mm ist, die sich ausgehend von dem proximalen Drahtabschnitt (13) des Führungsdrahts bis zu dem Anschluß des distalen Drahtkernsegments erstreckt.

4. Führungsdraht nach einem der vorhergehenden Ansprüche, bei dem:
(a) der flexible drehbare proximale Drahtabschnitt (13) einen Außendurchmesser von etwa 0,25 bis 1,00 mm besitzt, und
(b) das Zwischendrahtkernsegment einen verringerten Durchmesser von etwa 0,10 bis 0,50 mm besitzt, und
(c) das distale Drahtkernsegment eine verringerte Querschnittsfläche aufweist.

5. Führungsdraht nach Anspruch 4, bei dem das distale Drahtkernsegment zylinderförmig ist und einen Durchmesser von etwa 0,05 bis 0,15 mm aufweist.

6. Führungsdraht nach Anspruch 4, bei dem das distale Drahtkernsegment rechtekkig ist und Querschnittsabmessungen von 0,025 mm mal 0,075 mm aufweist.

7. Führungsdraht nach einem der Ansprüche 1 bis 3, bei dem:
(a) der flexible drehbare proximale Drahtabschnitt (13) einen Außendurchmesser von etwa 0,25 bis 0,50 mm aufweist, und
(b) das Zwischendrahtkernsegment einen verringerten Durchmesser von etwa 0,10 bis 0,20 mm besitzt, und
(c) das distale Drahtkernsegment einen verringerten Durchmesser von etwa 0,05 bis 0,13 mm aufweist.

8. Führungsdraht nach einem der vorhergehenden Ansprüche, bei dem eine schraubenförmige Platinspule (35), die um das proximale Ende des distalen Abschnitts (15) gewickelt ist, als ein Ankerpunkt für das proximale Ende der distalen Schraubenspule (27) und als der Ankerpunkt für das distale Ende des flexiblen Polymerschlauchmantels (19) dient, der das Zwischendrahtkernsegment und die schraubenförmige Bandspule (22) ummantelt.

## Patentansprüche (Patentansprüche für folgende(n) Vertragsstaat(en): DE, FR, GB, NL)

1. Katheter-Führungsdraht (10) zur Verwendung in dem Gefäßsystem eines Patienten, wobei der Führungsdraht in Kombination folgendes umfaßt:
(a) einen halbstarren drehbaren proximalen Drahtabschnitt (13),
(b) einen flexibleren Zwischenabschnitt (14), der aus einem Zwischendrahtkernsegment gebildet ist, das einen flexiblen Polymerschlauchmantel (19) aufweist, der das Zwischendrahtkernsegment ummantelt, und
(c) einen am meisten flexiblen distalen Endabschnitt (15), der aus einem distalen Drahtkernsegment mit einer schraubenförmigen Spule (27) gebildet ist, die das distale Endsegment abdeckt und diesem eine Knickfestigkeit verleiht,
wobei der Führungsdraht außerdem eine strahlenundurchlässige schraubenförmige Bandspule (22) umfaßt, die um das Zwischendrahtkernsegment zwischen dem Zwischendrahtkernsegment und dem flexiblen Polymerschlauchmantel (19) gewickelt ist und sich nur entlang dem Zwischendrahtkernsegment erstreckt, um die Drehmomentübertragung entlang dem Führungsdraht zu verbessern und um die Strahlenundurchlässigkeit des Zwischenabschnitts (14) gegenüber der des freiliegenden Zwischenkernsegments zu erhöhen, ohne daß dessen Steifigkeit wesentlich erhöht wird, und bei dem die Bandspule (22) aus einem strahlenundurchlässigen Metall gebildet ist, das aus der Gruppe ausgewählt wird, die aus Platin, Gold, Wolfram und deren Legierungen besteht.

2. Führungsdraht nach Anspruch 1, bei dem die Bandspule (22) eine straff gewickelte Spule mit einer Dicke von etwa 0,015 bis 0,050 mm und einer Breite von etwa 0,050 bis 0,130 mm ist, die sich ausgehend von dem proximalen Drahtabschnitt (13) des Führungsdrahts bis zu dem Anschluß des distalen Drahtkernsegments erstreckt.

3. Führungsdraht nach einem der vorhergehenden Ansprüche, bei dem:
(a) der flexible drehbare proximale Drahtabschnitt (13) einen Außendurchmesser von etwa 0,25 bis 1,00 mm besitzt, und
(b) das Zwischendrahtkernsegment einen verringerten Durchmesser von etwa 0,10 bis 0,50 mm besitzt, und
(c) das distale Drahtkernsegment eine verringerte Querschnittsfläche aufweist.

4. Führungsdraht nach Anspruch 3, bei dem das distale Drahtkernsegment zylinderförmig ist und einen Durchmesser von etwa 0,05 bis 0,15 mm aufweist.

5. Führungsdraht nach Anspruch 3, bei dem das distale Drahtkernsegment rechtekkig ist und Querschnittsabmessungen von 0,025 mm mal 0,075 mm aufweist.

6. Führungsdraht nach einem der Ansprüche 1 oder 2, bei dem:
(a) der flexible drehbare proximale Drahtabschnitt (13) einen Außendurchmesser von etwa 0,25 bis 0,50 mm aufweist, und
(b) das Zwischendrahtkernsegment einen verringerten Durchmesser von etwa 0,10 bis 0,20 mm besitzt, und
(c) das distale Drahtkernsegment einen verringerten Durchmesser von etwa 0,05 bis 0,13 mm aufweist.

7. Führungsdraht nach einem der vorhergehenden Ansprüche, bei dem eine schraubenförmige Platinspule (35), die um das proximale Ende des distalen Abschnitts (15) gewickelt ist, als ein Ankerpunkt für das proximale Ende der distalen Schraubenspule (27) und als der Ankerpunkt für das distale Ende des flexiblen Polymerschlauchmantels (19) dient, der das Zwischendrahtkernsegment und die schraubenförmige Bandspule (22) ummantelt.

## Revendications (Revendications pour l'(les) Etat(s) contractant(s) suivant(s): AT, BE, CH, DK, ES, GR, LI, IT, LU, MC, SE)

1. Fil de guidage de cathéter (10) destiné à être utilisé à l'intérieur du système vasculaire d'un patient, comprenant en combinaison :
(a) une section de fil proximale semi-rigide et pouvant subir un couple (13),
(b) une section intermédiaire plus flexible (14) formée à partir d'un segment de coeur de fil intermédiaire comportant un revêtement de tube polymère flexible (19) qui renferme le segment de coeur de fil intermédiaire, et
(c) une section d'extremité distale encore plus flexible (15) formée d'un segment de coeur de fil distal avec un enroulement hélicoïdal (27) qui recouvre le segment d'extrémité distale et qui communique une résistance à la flexion par compression axiale à celui-ci,
caractérisé en ce que le fil de guidage comprend de plus un enroulement en ruban hélicoïdal radio-opaque (22) enroulé autour du segment de coeur de fil intermédiaire entre le segment de coeur de fil intermédiaire et le revêtement de tube polymère flexible (19), et s'étendant le long du segment de coeur de fil intermédiaire uniquement de façon à améliorer la transmission de couple le long du fil de guidage et à augmenter la radio-opacité de la section intermédiaire (14) à une valeur supérieure à celle du segment de coeur intermédiaire nu sans augmenter sensiblement sa rigidité.

2. Fil de guidage selon la revendication 1, dans lequel l'enroulement en ruban (22) est formé à l'aide d'un métal radio-opaque sélectionné dans le groupe comprenant le platine, l'or, le tungstène et leurs alliages.

3. Fil de guidage selon la revendication 1 ou la revendication 2, dans lequel l'enroulement en ruban (22) est un enroulement enroulé étroitement avec une épaisseur d'environ 0,015 à 0,050 mm et une largeur d'environ 0,050 à 0,130 mm, qui s'étend de la section de fil proximale (13) du fil de guidage à la jonction du segment de coeur de fil distal.

4. Fil de guidage selon l'une quelconque des revendications précédentes, dans lequel :
(a) la section de fil proximale flexible pouvant subir un couple (13) a un diamètre extérieur compris entre environ 0,25 et 1,00 mm, et
(b) le segment de coeur de fil intermédiaire a un diamètre réduit compris entre environ 0,10 et 0,50 mm, et
(c) le segment de coeur de fil distal a une surface de section transversale réduite.

5. Fil de guidage selon la revendication 4, dans lequel le segment de coeur de fil distal est cylindrique et a un diamètre compris entre environ 0,05 et 0,15 mm.

6. Fil de guidage selon la revendication 4, dans lequel le segment de coeur de fil distal est rectangulaire avec des dimensions de section transversale de 0,025 mm sur 0,075 mm.

7. Fil de guidage selon l'une quelconque des revendications 1 à 3, dans lequel :
(a) la section de fil proximale flexible pouvant subir un couple (13) a un diamètre extérieur compris entre environ 0,25 et 0,50 mm, et
(b) le segment de coeur de fil intermédiaire a un diamètre réduit compris entre environ 0,10 et 0,20 mm, et
(c) le segment de coeur de fil distal a un diamètre réduit compris entre environ 0,05 et 0,13 mm.

8. Fil de guidage selon l'une quelconque des revendications précédentes, dans lequel un enroulement hélicoïdal en platine (35) enroulé autour de l'extrémité proximale de la section distale (15) joue le rôle de point d'ancrage pour l'extrémité proximale de l'enroulement hélicoïdal distal (27) et de point d'ancrage pour l'extrémité distale du revêtement de tube polymère flexible (19) qui renferme le segment de coeur de fil intermédiaire et l'enroulement en ruban hélicoïdal (22).

## Revendications (Revendications pour l'(les) Etat(s) contractant(s) suivant(s): DE, FR, GB, NL)

1. Fil de guidage de cathéter (10) destiné à être utilisé à l'intérieur du système vasculaire d'un patient, comprenant en combinaison :
(a) une section de fil proximale semi-rigide et pouvant subir un couple (13),
(b) une section intermédiaire plus flexible (14) formée à partir d'un segment de coeur de fil intermédiaire comportant un revêtement de tube polymère flexible (19) qui renferme le segment de coeur de fil intermédiaire, et
(c) une section d'extrémité distale encore plus flexible (15) formée d'un segment de coeur de fil distal avec un enroulement hélicoïdal (27) qui recouvre le segment d'extrémité distale et qui communique une résistance à la flexion par compression axiale à celui-ci,
dans lequel le fil de guidage comprend de plus un enroulement en ruban hélicoïdal radio-opaque (22) enroulé autour du segment de coeur de fil intermédiaire entre le segment de coeur de fil intermédiaire et le revêtement de tube polymère flexible (19), et s'étendant le long du segment de coeur de fil intermédiaire uniquement de façon à améliorer la transmission de couple le long du fil de guidage et à augmenter la radio-opacité de la section intermédiaire (14) à une valeur supérieure à celle du segment de coeur intermédiaire nu sans augmenter sensiblement sa rigidité, et dans lequel l'enroulement en ruban (22) est formé en un métal radio-opaque sélectionné dans le groupe comprenant le platine, l'or, le tungstène et leurs alliages.

2. Fil de guidage selon la revendication 1, dans lequel l'enroulement en ruban (22) est un enroulement enroulé étroitement avec une épaisseur d'environ 0,015 à 0,050 mm et une largeur d'environ 0,050 à 0,130 mm, qui s'étend de la section de fil proximale (13) du fil de guidage à la jonction du segment de coeur de fil distal.

3. Fil de guidage selon l'une quelconque des revendications précédentes, dans lequel :
(a) la section de fil proximale flexible pouvant subir un couple (13) a un diamètre extérieur compris entre environ 0,25 et 1,00 mm, et
(b) le segment de coeur de fil intermédiaire a un diamètre réduit compris entre environ 0,10 et 0,50 mm, et
(c) le segment de coeur de fil distal a une surface de section transversale réduite.

4. Fil de guidage selon la revendication 3, dans lequel le segment de coeur de fil distal est cylindrique et a un diamètre compris entre environ 0,05 et 0,15 mm.

5. Fil de guidage selon la revendication 3, dans lequel le segment de coeur de fil distal est rectangulaire avec des dimensions de section transversale de 0,025 mm sur 0,075 mm.

6. Fil de guidage selon l'une ou l'autre des revendications 1 et 2, dans lequel :
(a) la section de fil proximale flexible pouvant subir un couple (13) a un diamètre extérieur compris entre environ 0,25 et 0,50 mm, et
(b) le segment de coeur de fil intermédiaire a un diamètre réduit compris entre environ 0,10 et 0,20 mm, et
(c) le segment de coeur de fil distal a un diamètre réduit compris entre environ 0,05 et 0,13 mm.

7. Fil de guidage selon l'une quelconque des revendications précédentes, dans lequel un enroulement hélicoïdal en platine (35) enroulé autour de l'extrémité proximale de la section distale (15) joue le rôle de point d'ancrage pour l'extrémité proximale de l'enroulement hélicoïdal distal (27) et de point d'ancrage pour l'extrémité distale du revêtement de tube polymère flexible (19) qui renferme le segment de coeur de fil intermédiaire et l'enroulement en ruban hélicoïdal (22).
